# EUROPEAN PATENT APPLICATION

(11) **EP 1 927 337 A1**
(43) Date of publication of application: **04.06.2008**
(21) Application number: 06024374.8
(22) Date of filing: 24.11.2006
(51) Int. Cl.: A61K 8/11, A61K 8/26, A61K 8/29, A61K 8/41, A61Q 5/12

(54) **Conditioning composition for keratin fibres**

(71) Applicant: KPSS-Kao Professional Salon Services GmbH, 64297 Darmstadt (DE)
(72) Inventor: Hoffmann, Martin, 64673 Zwingenberg (DE); Tietjen, Ilka, 69207 Sandhausen (DE)

(57) **Abstract**

**SUMMARY**

The present invention relates to a conditioning and shine enhancing composition for keratin fibres especially human hair. Particularly, the subject of the present invention is a composition comprising at least one cationic surfactant at a concentration of 0.01 to 10% by weight, and at least one colour effect pigment consisting of synthetic mica coated with metal oxide or oxides and having a volume particle size distribution of 1 to 750 µm at a concentration of 0,01 to 10% by weight, calculated to total composition, which gives hair shimmery shine.

## Description

The present invention relates to a conditioning and shine enhancing composition for keratin fibres especially human hair.

Hair conditioning compositions have widely been used for improving primarily combability of hair and furthermore enhancing smoothness, elasticity and shine, Many type of conditioners have been found on the market varying from emulsions, which are generally rinsed of from hair after application and certain period of processing time, to low viscosity lotions used without rinsing off after application. Hair shine improvement has been one of the main areas of development, Hair shine is very much related to the surface structure of hair and this varies very much with the degree of damage either by environmental effects or chemical treatment of hair such as permanent shaping or oxidative colouring. Although consumers with healthy non-damaged hair are generally satisfied with hair shine, shine of damaged hair is usually found to be unsatisfactory, There have been studies aiming improving shine of especially damaged hair.

The inventors of the present invention have surprisingly found out that a composition comprising at least one cationic surfactant and a colour effect pigment of synthetic mica coated with metal oxide or oxides improves shine and conditions hair excellently in terms of combability, elasticity, smoothness and softness.

Accordingly the subject of the present invention is an aqueous composition comprising at least one cationic surfactant at a concentration of 0.01 to 10% by weight, and at least one colour effect pigment consisting of synthetic mica coated with metal oxide or oxides and having a volume particle size distribution in the range of 1 to 750 µm at a concentration of 0.01 to 10% by weight, calculated to total composition.

Use of synthetic mica coated with metal oxide or oxides mainly in decorative cosmetics is disclosed in an international patent application of Sun Chemical Corporation published with a number WO 2005/065632 A1. In the document synthetic mica and coated synthetic mica with at least one metal oxide or oxides is disclosed in detail, the content of the document Is included herewith by reference. Suitable metal oxide or oxides for coating synthetic mica are titanium dioxide, chromium oxide, ferric oxide or mixtures thereof, In the present invention the preferred is synthetic mica coated with titanium dioxide. Such materials are commercially available from Sun Chemical Corporation and known with their INCI names Synthetic Fluorphologopite.

The particle size distribution of synthetic mica coated with a metal oxide or oxides is in the range of 1 to 750 µm, preferably 1 to 250 µm, more preferably 1 to 100 µm and most preferably 20 to 95 µm. The particle sizes referred are relating to the volume particle size distribution meaning that particles found in the coated synthetic mica having volume particle size in the given ranges.

Concentration of synthetic mica coated with at least metal oxide or oxides is from 0.001 to 10%, preferably 0,05 to 7.5%, more preferably 0.1 to 5°/o and most preferably 0.25 to 2.5% by weight calculated to total composition.

Compositions of the present invention comprise at least one cationic surfactant at a concentration of 0.01 to 10%, preferably 0.05 to 7.5%, more preferably 0.1 to 5% by weight calculated to total composition.

As a rule any cationic surfactant is suitable for the compositions of the present invention. With the term cationic surfactant it is meant that the surfactant carries a cationic charge when used in the compositions. In other words, compounds having no cationic charge but when added into the compositions protonate and therewith become cationic are also included within the definition of cationic surfactant. An example to such may be stearyldimethylamine and PEG-2 Cocamine are as a compound not carrying a cationic charge but when used in a composition having acidic pH becomes cationic by protonation.

Preferably at least one cationic surfactant is selected from the compounds with the general formula 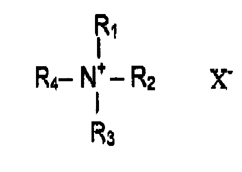 where R₁s a saturated or unsaturated, branched or non-branched alkyl chain with 8-22 C atoms or

R₅CO NH (CH₂)ₙ

where R₅ is saturated or unsaturated, branched or non-branched alkyl chain with 7-21 C atoms and n has typical value of 0 - 4 or

R₅CO O (CH₂)n

where R₆ is saturated or unsaturated, branched or non-branched alkyl chain with 7-21 C atoms and n has typical value of 0 - 4, and
R₂ is a hydrogen, saturated or unsaturated, branched or non-branahed alkyl chain with 1-22 C atoms or

R₅CO NH (CH₂)n

where R₅ is saturated or unsaturated, branched or non-branched alkyl chain with 7-21 C atoms and n has typical value of 0 - 4 or

R₆ CO O (CH₂)n

where R₆ is saturated or unsaturated, branched or non-branched alkyl chain with 7-21 C atoms and n has typical value of 0 - 4,
and R₃ and R₄ are independent from each other H or lower alkyl chain with 1 to 4 carbon atoms or ethoxy or propoxy group with number of ethoxy or propoxy groups varying in the range of 0 to 4, and X is chloride, bromide or methosulfate.

Suitable cationic surfactants and or conditioning agents are, for example, long-chain quaternary ammonium compounds which can be used alone or in admixture with one another, such as cetyl trimethyl ammonium chloride, myristoyl trimethyl ammonium chloride, behentrimonium chloride, trimethyl cetyl ammonium bromide, stearyl trimethyl ammonium chloride, dimethyl stearyl ammonium chloride, dimethyl dihydrogenated tallow ammonium chloride, stear trimonium chloride, stearamidopropyldimethylamoonium chloride, dipalmitoyl dimonium chloride, distearyl dimethyl ammonium chloride, stearamidopropyl trimonuim chloride.

Further examples to the cationic surfactants are so called esterquats available on the market, for example, under the trade names "Schercocquat^{®}", "Dehyquart^{®} F30" and "Tetranyl^{®}". Still further examples are so called amidoquatsAgain available on the market, for example, under the trade name "INCROQUAT^{ȧ} HO" or "OCS".

Compositions of the present invention can be in the form of a thin liquid, emulsion, thickened liquid or gel, In the case that the compositions are in the form of a thin liquid, it may be that metal oxide or oxides coated synthetic particles are precipitated or flocculated so that should be used after uniformly distributing the particles in the composition by agitation by for example shaking. In such a case it should also be possible to mix metal oxide or oxides coated synthetic mica particles prior to application onto keratin fibres with a composition comprising at least one cationic surfactant. Emulsion, thickened liquid and gel compositions are preferred within the meaning of the present invention.

Accordingly, another subject of the present invention is process for treating keratin fibres especially human hair wherein synthetic mica coated with metal oxide or oxides is added into the composition comprising at least one cationic surfactant prior to application onto hair.

With the term thickened liquid, it is meant that the compositions comprise additionally a thickening agent.

With the term gel it is meant that the compositions comprise additionally a gelling agent and the gelling agent is a polymer forming a shear thinning gel.

The thickening agents include any polymer either natural or synthetic thickening aqueous composition, Examples are cellulose and its derivatives such as hydroxyethylcellulose, guar and its derivatives such as hydroxypropyl guar. In the selection of the thickening gels compatibility with cationic surfactant should be carefully examined.

The gelling agents include polymers either synthetic or natural forming shear thinning compositions. Examples to the natural polymers are xanthan gum and its derivatives. Synthetic shear thinning polymers may be those of acrylate polymers wherein compatibility with cationic surfactant should carefully be examined prior to use.

Concentration of the thickening and/or gelling agents should be in the range of 0.05 to 5%, preferably 0.1 to 2,5% by weight calculated to total content. It should also be noted that gelling and thickening polymers can be used together.

Another preferred form is oil in water (O/VV) emulsion. Emulsions according to the present invention preferably comprise at least one fatty alcohol with linear of branched alkyl chain. Suitable ones are fatty alcohols having 12 to 22 C atoms in its alkyl chain. Examples are myristyl alcohol, cetyl alcohol, stearyl alcohol, behenyl alcohol and their mixtures. Preferred are cetyl, stearyl and behenyl alcohol and their mixtures i.e. cetearyl alcohol. Fatty alcohols may be included into the compositions of the present invention at a concentration of 0,1 to 20%, preferably 0.5 to 15% and more preferably 1 to 10% by weight calculated to total composition.

Emulsions should also comprise at least one emulsifier. It should be noted that quaternary ammonium compounds with single alkyl chain mentioned above are preferred as emulsifiers as well.

In addition to the cationic surfactants with single alkyl chain, additional emulsifier can be incorporated into the compositions. These additional emulsifiers are surface active substances such as non-ionic, amphoteric or zwitterionic and anionic compounds. Because of the compatibility issues of the anionic surfactants and cationic surfactants mentioned above, anionic surfactants are less suitable and therefore their compatibility should carefully be examined. Otherwise, the compositions of the present invention preferably should substantially be free of anionic surfactants, Preferred emulsifiers are cationic, non-ionic, amphoteric or zwitterionic surfactants. The most preferred additional emulsifiers are non-ionic surfactants.

Suitable non-ionic surfactants are alkyl polyglucosides of the general formula

R₇ - O - (R₈O)ₙO - Zₓ

wherein R₇ is an alkyl group with 8 to 18 carbon atoms, R₈ is an ethylene or propylene group, Z is a saccharide group with 5 to 6 carbon atoms, n is a number from 0 to 10 and x is a number between 1 and 5. Examples are decyl polyglucoside, cocoyl polyglucoside both are commercially available.

Further nonionic surfactant components are, for example, long-chain fatty acid mono- and dialkanolamides, such as coco fatty acid monoethanolamide and myristic fatty acid monoethanolamide.

Further additionally useful nonionic surfactants are, for example, the various sorbitan esters, such as polyethylene glycol sorbitan stearic acid ester, fatty acid polyglycol esters or poly-condensates of ethyleneoxide and propyleneoxide, as they are on the market, for example, under the trade name "Pluronics^{R}".

Further nonionic surfactants as emulsifiers useful in the compositions according to invention are C₁₀-C₂₂-fatty alcohol ethoxylates. Especially suited are C₁₀-C₂₂-fatty alcohol ethers, the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16":

The average degree of ethoxylation thereby ranges between about 2.5 and about 25, preferably about 10 and about 20.

Among the non-ionic surfactants mentioned above fatty alcohol ethoxylates are the most preferred ones. Above mentioned non-ionic surfactants can also be used as mixture of one category such as several ethoxylated fatty alcohols or several categories such as mixture of alkyl polyglucoside and ethoxylated fatty alcohol.

As further surfactant component as emulsifier, the compositions according to the invention can also contain amphoteric or zwitterionic surfactants. Useful as such are in particular the various known betaines such as alkyl betaines, fatty acid amidoalkyl betaines and sulfobetaines, for example, lauryl hydroxysulfobetaine; long-chain alkyl amino acids, such as cocoaminoacetate, cocoaminoproplonate and sodium cocoamphopropionate and -acetate.

Additional emulsifier content of the compositions according to present invention is in the range of 0.05 to 10%, preferably 0.1 to 7.5% and more preferably 0.25 to 5% by weight calculated to total composition.

Compositions of the present invention can comprise additional hair conditioning compounds such as oils, cationic polymers, non-ionic substances. Oils as conditioners according to the present invention are selected from silicone oils either volatile or non-volatile, natural and synthetic oils, Among silicone oils those can be added to the compositions include dimethicone, dimethiconol, polydimethylsiloxane, DC fluid ranges from Dow Coming, cyclosiloxanes such as DC 245, arylated silicones such as phenyltrimethicone available from Dow Coming under trade name DC 556. Synthetic oils include mineral oil such as paraffin oil and petrolatum.

Natural oils suitable are such as olive oil, almond oil, avocado oil, weizenkeim oil, ricinus oil, coconut oil, palm oil, sesame oil, peanut oil, whale oil, sunflower oil, peach kernel oil, wheat germ oil, macadamia nut oil, night primrose oil, jojoba oil, castor oil, or soya oil, lanolin and the derivatives thereof.

Lipophilic oily compounds such as fatty acid esters are as well suitable for the composition of the present invention. Those are such as isopropyl myristate, palmitate, stearate and isostearate, oleyl oleate, isocetyl stearate, hexyl laurate, dibutyl adipate, dioctyl adipate, myristyl myristate, oleyl erucate, cetyl palmitate, etc.

Non-ionic conditioning agents may be polyols such as glycerin, glycol and derivatives, polyethyleneglycoles known with trade names Carbowax PEG from Union Carbide and Polyox WSR range from Amerchol, polyglycerin, polyethyleneglycol mono or di fatty acid esters having general formula

R₉ CO (OCH₂CH₂)ₙ OH

Re CO (OCH₂CH₂)n O OC R₁₀ where R₉ and R₁₀ are independent from each other saturated, unsaturated or branched or non-branched alkyl chain with 7 to 21 C atoms and n is typically 2-100. Typical concentration range for any of the additional conditioners mentioned above other than cationic conditioning compounds can be in the range of 0,01 to 15% by weight, preferably 0.05 -10% by weight, more preferably 0.1- 5% by weight calculated to the total composition.

Composition of the present invention comprises cationic polymers as conditioning agents. Those are cationic cellulose type polymers know as Polymer JR type from Amerchol such as Polyquaternium 10 or cationic guar gum known with trade name Jaguar from Rhône-Poulenc and chemically for example Guar hydroxypropyl trimonium chloride. Furthermore, chitosan and chitin can also be included in the compositions as cationic natural polymers.

Furthermore, it has especially been found suitable those cationic polymers known with their CTFA category name Polyquaternium, Typical examples of those Polyquaternium 6, Polyquaternium 7, Polyquaternium 10, Polyquaternium 11, Polyquaternium 18, Polyquaternium 22 and Polyquaternium 28, Polyquaternium 30, Polyquaternium 37, Polyquaternium 36, Polyquaternium 46, Polyquaternium 67.

As well those polymers known with their CTFA category name Quaternium are suitable. Those are for example Quaternium-8, Quaternium-14, Quaternium-15, Quaternium-18, Quaternium-22, Quaternium-24, Quaternium-26, Quaternium-27, Quaterniurn-30, Quaternium-33, Quaternium-53, Quaternium-60, Quaternium-61, Quaternium-72, Quaternium-78, Quaternium-80, Quaternium-81, Quaternium-81, Quaternium-82, Quaternium-83 and Quaternium-84.

In this context, reference is also made to the cationic polymers disclosed in DE 25 21 960, 28 11 010, 30 44 738 and 32 17 059, as well as to the products described in EP-A 337 354 on pages 3 to 7. It is also possible to use mixtures of various cationic polymers.

The cationic polymers also include the quaternized products of graft polymers from organopolysiloxanes and polyethyl oxazolines described in EP-A 524 612 and EP-A 640 643.

The compositions according to the present invention can also comprise further agents, such as protein hydrolyzates and polypeptides, e.g. keratin hydrolyzates, collogen hydrolyzates of the type "Nutrilan" or elastin hydrolyzates, as well as, in particular vegetable, optionally cationized protein hydrolyzates, for example "Gluadin".

Additional natural plant extracts can as well form part of the compositions of the present invention. Those are incorporated usually in an amount of about 0.01 % to about 10 %, preferably 0.05 % to 7.5 %, in particular 0.1 % to 5 % by weight, calculated as dry residue thereof to the total composition. Suitable aqueous (e.g. steam-distiled) alcoholic or hydro-alcoholic plant extracts known per se are in particular extracts from leaves, fruits, blossoms, roots, rinds or stems of aloe, pineapple, artichoke, arnica, avocado, valerian, bamboo, green tea, blue lotus flower, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, cocoanut, cornflower, time blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc,

Suitable trade products are, for example, the various "Extrapone" products and "Herbasol^{R} ". Extracts and the preparation thereof are also described in "Hagers Handbuch der pharmazeutischen Praxis", 4th Ed.

The compositions may contain organic solvents such as ethanol, propanol, isopropanol, benzyl alcohol, benzyloxyethanol, alkylene carbonates such as ethylene carbonate and propylene carbonate, phenoxyethanol, butanol, isobutanol, cyclohexane, cyclohexanol, hexyleneglycol, ethylenecarbonate, ethyleneglycol monoethylether, ethylene glycol monobutyl ether, ethylene glycol monophenyl ether, 1-phenylethylalcohol, 2-phenylethylalcohol, o-methoxyphenol. Concentration of organic solvents in the composition should not exceed 5% by weight. It should be noted that penetration enhancers are useful for both cleansing and after shampoo conditioning preparations. It is obvious that the concentration in the cleansing compositions is usually lower than in the conditioning preparations,

Compositions of the present invention can comprise W filters either for stabilization of the product colour or for protection of hair from environmental influences such as loss of elasticity, loss of hair colour (bleaching effect of sun light). The UV-absorbing substance is preferably selected from the following compounds: 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2.4-dihydroxybenzophenone, 2,2'.4,4'-tetrahydroxy- benzophenone, 2-hydroxy-4-methoxybenzophenone and its 5-sulfonic acid or the sodium salt thereof, 2.2'-dihydroxy-4.4'-dimethoxybenzophenone, 2-hydroxy-5-chlorobenzophenone, 2.2'-dihydroxy-4-methoxybenzophenone, 2.2'-dihydroxy-4.4'-dimethoxy-5.5'-disulfobenzophenone or the sodium salt thereof, 2-hydroxy-4-octyloxybenzophenone, 2-hydroxy-4-mothoxy-4'-methylbenzophenone, 3-benzyl-idenecampher, 3-(4'-sulfo)-benzylidenebomane-2-one and the salts thereof and/or 3-(4'-methyl benzylidene)-DL-campher, polysilicone-1 5. The preferred amount of the UV-absorber ranges from about 0.01 % to 2.5%, more preferably from 0.05 % to 1 % by weight, calculated to the total composition.

The compositions of the present Invention can comprise hair-restructuring agents. The hair restructuring agents preferred are especially the ones disclosed in the German patent DE 197 51 550 C2. Namely they are ceramide type of compounds, fatty acids and phytosterol or their mixtures.

Preferred ceramide compound is cetyl-PG-hydroxyethylpalmitamide.

Preferred fatty acids are those with 10 to 24 carbon atoms and especially with 16 to 24 carbon atoms.

Sterols,especially the phytosterols, are as well preferred hair restructuring agents as disclosed in the above mentioned german patent. Especially preferred ones are of plant origin for example ergosterol, sitosterol, stigmasterol, fucosterol, brassicasterol, fungisterol, campesterol, zymosterol, ascosterol, cerevisterol, episterol, faecosterol, spinasterol. Among those phytosterols, the ones found in "Avocadin" which is the unsaponified fraction of the avocado oil is more preferred.

The concentration of ceramide in the compositions of the present Invention can be in the range of 0.01 to 2% and especially 0.01 to 1% by weight calculated to the total weight of the composition. The fatty acids may be contained at a level of 0.01 to 2.5% and especially 0.01 to 1% by weight calculated to the total weight of the composition. Phytosterol concentration of the conditioners is less than 1% and preferably in the range of 0.01 to 0.5% by weight calculated to the total weight of the composition, It should be noted without limiting the use of those ingredients the effect of those hair restructuring ingredients is especially elevated when used in combination with penetration enhancers.

The pH of the compositions according to the invention is in the range of 2 to 7, preferably 3 to 6, more preferably 3 to 5. For adjusting the pH of the said compositions, following ingredients can be used; Organic acids such as citric acid, lactic acid, tartaric acid, malic acid, maleic acid, fumaric acid, levulinic acid, butyric acid and hydroxy butyric acids, valeric acid, oxalic acid, succinic acid, mandelic acid, glycolic acid, glucuronic acid, propionic acid, salicylic acid or acetic acid or inorganic acids such as hydrochloric acid, phosphoric acid, sulphuric acid, nitric acid. Concentration of the organic and/or inorganic acids or their mixtures should be chosen in a way that composition reaches the desired pH value as given above. Typically concentration for acids can be 0.01 - 3% by weight, preferably 0.05 - 2% by weight, more preferably 0.05 - 1,5% by weight calculated to the total composition. The pH of the composition can also be adjusted to the required pH by using alkaline solution such as sodium hydroxide, ammonium hydroxide, potassium hydroxide or their salts with those acids mentioned above in the case that at the selected acid concentration pH of the composition is lower than that of the aimed value.

Furthermore compositions of the present invention can comprise all substances customarily found in such preparations. Examples of such substances are complexing agents, dyestuffs preferably non-substantive for colouring composition, preservatives, fragrances, etc.

Compositions of the present invention are used as a rinse off conditioners and usually used after cleansing hair, On the other hand usage without previous cleansing should not be excluded, Accordingly, process for conditioning keratin fibres especially human hair, especially enhancing shine consists of washing hair, preferably with a cleansing composition and then applying a composition comprising at least one cationic surfactant at a concentration of 0.01 to 10% by weight, and at least one colour effect pigment consisting of synthetic mica coated with metal oxide or oxides and having a particle size distribution of 1 to 750 µm at a concentration of 0.01 to 10% by weight, calculated to total composition, onto hair and after processing for 30 sec to 30 min, preferably 1 min to 15 min and more preferably 3 minute to 10 min at ambient temperature or at a temperature not exceeding 40°C, rinsed off.

Following examples are to illustrate the invention but not to limit.

### Example 1

| | % by weight |
|---|---|
| Cetrimonium chloride | 2.0 |
| Synthetic fluorphologopite* | 1.0 |
| Citric acid/Sodium hydroxide | q.s. to pH 4.0 |
| Fragrance | 0.2 |
| Solubilizer** | 0,2 |
| Water | to 100 |

| | |
|---|---|
| *: Synthetic fluorphologopite used is commercially available from Sun Chemical Corporation under the trade name SunShine Glitter White with a particle size distribution in the range of 20 to 95 µm, **: any solubilizer may be used preferred are those ethoxylated ricinus oil, preferably hydrogenated ricinus oil. In the above example and other examples below where necessary PEG-60 hydrogenated ricinus oil is used preferably at a weight ratio of fragrance to solubilizer 1:1. | |

The above composition was prepared by combining cetrimonium chloride (a commercially available 25% by weight solution was used) with remaining water. Afterwards fragrance and solubilizer was combined, solubilizer was heated slightly to melt before, and added to the solution of cetrimonium chloride. Finally synthetic fluorphologopite was dispersed and pH was adjusted to 4.0.

The above composition was tested in a half side test with 10 consumers having shoulder length hair. Before application of the above composition, hair was washed with a commercially available shampoo. Afterwards to the half side 5 g of the above composition was applied and the other half was left untreated. After processing time of 5 min at ambient temperature the composition was rinsed off from hair. The hair was towel dried and dried with a hair drier. The hair was combable and had excellent shimmery shine, The composition was shaken to homogeneity prior to application. Interestingly spraying is also possible application way.

### Example 2

| | % by weight |
|---|---|
| Cetearyl alcohol | 10.0 |
| Behentrimonium chloride | 2.0 |
| Synthetic fluorphologopite* | 1.0 |
| Citric acid/Sodium hydroxide | q.s. to pH 4.0 |
| Fragrance | 0.4 |
| Water | to 100 |

| | |
|---|---|
| *: Synthetic fluorphologopite used is commercially available from Sun Chemical Corporation under the trade name SunShine Glitter White with a particle size distribution in the range of 20 to 95 µm. | |

Above composition was prepared first by emulsifying cetearyl alcohol and behentrimonium chloride at a temperature of approximately 75°C in part of water. Afterwards the composition was cooled down and remaining water was added which was followed by addition of fragrance and Synthetic fluorphologopite pre-dispersed in a small portion of water. Finally pH was adjusted.

For comparative purposes the same composition but not comprising Synthetic fluorphologopite was also produced.

The above composition was tested in a half side test against the comparative composition without Synthetic filuorphologopite in the same way as in Example 1 with 10 consumers having shoulder length hair. Comments from the consumer were both side feels soft and combable but the side treated with the inventive composition has significantly more shimmery shine than the side treated with the comparative composition. The preference was 1010,

### Example 3

| | % by weight |
|---|---|
| Cetearyl alcohol | 10 |
| Behentrimonium chloride | 2.0 |
| Synthetic fluorphologopite* | 1.0 |
| Citric acid/Sodium hydroxide | q.s. to pH 4.0 |
| Fragrance | 0.4 |
| Water | to 100 |

| | |
|---|---|
| *: Synthetic fluorphologopite used is commercially available from Sun Chemical Corporation under the trade name SunShine Ultra Glitter White with a particle size distribution in the range of 95 to 730 µm. | |

The composition was prepared in the similar way as in Example 2.

In a half side test similar results were obtained as in the Example 2.

Similar results were observed with the compositions below.

### Example 4

| | % by weight |
|---|---|
| Cetearyl alcohol | 10 |
| Behentrimonium chloride | 2.0 |
| Synthetic fluorphologopite* | 1.0 |
| Citric acid/Sodium hydroxide | q.s. to pH 4.0 |
| Fragrance | 0.4 |
| Water | to 100 |

| | |
|---|---|
| *: Synthetic fluorphologopite used is commercially available from Sun Chemical Corporation under the trade name SunShine Super Glitter White with a particle size distribution in the range of 40 to 250 µm. | |

### Example 5

| | % by weight |
|---|---|
| Behentrimonium chloride | 2.0 |
| Synthetic fluorphologopite* | 1.0 |
| Hydroxyethylceitutose | 1.0 |
| Citric acid/Sodium hydroxide | q.s. to pH 4.0 |
| Fragrance | 0.4 |
| Solubilizer as in Example 1 | 0.4 |
| Water | to 100 |

| | |
|---|---|
| *: synthetic fluorphologopite used is commercially available from Sun Chemical Corporation under the trade name SunShine Glitter White with a particle size distribution in the range of 20 to 95 µm, | |

### Example 6

| | % by weight |
|---|---|
| Behentrimonium chloride | 1.5 |
| Synthetic fluorphologopite* | 1.0 |
| Polyquatemlum-10 | 0.7 |
| Hydroxyethylcellulose | 0.5 |
| Citric acid/Sodium hydroxide | q.s. to pH 3.5 |
| Fragrance | 0.4 |
| Solubilizer as in Example 1 | 0.4 |
| Water | to 100 |

| | |
|---|---|
| *: Synthetic fluorphologopite used is commercially available from Sun Chemical Corporation under the trade name SunShine Glitter White with a particle size distribution in the range of 20 to 95 µm. | |

### Example 7

| | % by weight |
|---|---|
| Behentrimonium chloride | 1.5 |
| Synthetic fluorphologopite* | 1.0 |
| Polyquaternium 37 | 0.5 |
| Citric acid/Sodium hydroxide | q.s, to pH 4,5 |
| Fragrance | 0.4 |
| Solubilizer as in Example 1 | 0.4 |
| Water | to 100 |

| | |
|---|---|
| *: Synthetic fluorphologopite used is commercially available from Sun Chemical Corporation under the trade name SunShine Glitter White with a particle size distribution in the range of 20 to 95 µm. | |

### Example 8

| | % by weight |
|---|---|
| Behentrimonium chloride | 1.5 |
| Synthetic fluorphotogopite* | 1.0 |
| Dimethicone | 0.5 |
| Hydroxypropyl guar | 0.8 |
| Citric acid/Sodium hydroxide | q.s. to pH 4.5 |
| Fragrance | 0.4 |
| Solubilizer as in Example 1 | 0.4 |
| Water | to 100 |

| | |
|---|---|
| *: Synthetic fluorphologopite used is commercially available from Sun Chemical Corporation under the trade name SunShine Glitter White with a particle size distribution in the range of 20 to 95 µm. | |

## Claims

1. Aqueous composition for conditioning keratin fibres especially for human hair **characterised in that** it comprises at least one cationic surfactant at a concentration of 0.01 to 10% by weight, and at least one colour effect pigment consisting of synthetic mica coated with metal oxide or oxides and having a volume particle size distribution of 1 to 750 µm at a concentration of 0.01 to 10% by weight, calculated to total composition.

2. Composition according to claim 1 **characterised in that** at least one cationic surfactant is selected form a compound of the general formula 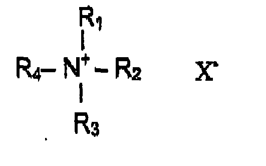 where R₁s a saturated or unsaturated, branched or non-branched alkyl chain with 8-22 C atoms or
R₅CO NH (CH₂)n
where R₅ is saturated or unsaturated, branched or non-branched alkyl chain with 7-21 C atoms and n has typical value of 0 - 4 or
R₆CO O (CH₂)ₙ
where R₆ is saturated or unsaturated, branched or non-branched alkyl chain with 7-21 C atoms and n has typical value of 0 - 4, and
R₂ is a hydrogen, saturated or unsaturated, branched or non-branched alkyl chain with 1-22 C atoms or
R₅CO NH (CH₂)ₙ
where R₆ is saturated or unsaturated, branched or non-branched alkyl chain with 7-21 C atoms and n has typical value of 0 - 4 or
R₆ CO O (CH₂)ₙ
where R₆ is saturated or unsaturated, branched or non-branched alkyl chain with 7-21 C atoms or ethoxy or propoxy group with number of ethoxy or propoxy groups varying in the range of 0 to 4 and n has typical value of 0 - 4,
and R₃ and R₄ are independent from each other H or lower alkyl chain with 1 to 4 carbon atoms, and X is chloride, bromide or methosulfate.

3. Composition according to claims 1 and 2 **characterised in that** at least one colour effect pigment has a volume particle size distribution in the range of 20 to 95 µm.

4. Composition according to any of the preceding claims **characterised in that** it comprises at least one gelling agent and/or at least one thickening agent.

5. Composition according to any of the preceding claims **characterised in that** it comprises at least one fatty alcohol.

6. Composition according to any of the preceding claims **characterised in that** it comprises at least one organic solvent.

7. Composition according to any of the preceding claims **characterised in that** it comprises at least one UV filter.

8. Composition according to any of the preceding claims **characterised in that** it comprises at least one additional conditioning agent selected from oils, cationic polymers and non-ionic compounds

9. Process for conditioning keratin fibres especially human hair **characterised in that** after washing with a cleansing composition or wetting, an aqueous composition according to claims 1 to 8 is applied and after processing period of 30 sec to 30 min rinsed off.

10. Use of an aqueous composition according to claims 1 to 8 for enhancing shine of keratin fibres especially human hair.
